# EUROPEAN PATENT SPECIFICATION

(11) **EP 2 784 159 B1**
(45) Date of publication and mention of the grant of the patent: **09.05.2018**
(21) Application number: 12852194.5
(22) Date of filing: 08.11.2012
(51) Int. Cl.: C12N 15/11, C12N 15/85, C12N 15/33, C12N 5/10

(54) **HEPATITIS B VIRUS-DERIVED CIS-REGULATORY ELEMENT AND USE THEREOF**
CIS-REGULIERENDES ELEMENT AUS HEPATITIS-B-VIRUS UND DESSEN VERWENDUNG
ÉLÉMENT CIS-RÉGULATEUR DÉRIVÉ DU VIRUS DE L'HÉPATITE B ET SON UTILISATION

(30) Priority: 25.11.2011 KR 20110124053
(43) Date of publication of application: 01.10.2014
(73) Proprietor: SNU R&DB Foundation, Seoul 151-742 (KR)
(72) Inventor: KIM, Bum-Joon, Seoul 135-110 (KR); LEE, Seoung-Ae, Seoul 110-530 (KR)
(74) Representative: Ter Meer Steinmeister & Partner
(86) International application number: PCT/KR2012/009376
(87) International publication number: WO 2013/077573

(56) References cited:
- WO-A1-95/25785
- KR-B1- 100 452 718
- DATABASE WPI Week 200848 Thomson Scientific, London, GB; AN 2008-H54103 XP002740693, -& KR 100 758 727 B1 (UNIV SEOUL NAT IND FOUND) 14 September 2007 (2007-09-14)
- CHAUDHURI ET AL: "Occult hepatitis B virus infection in chronic liver disease: Full-length genome and analysis of mutant surface promoter", GASTROENTEROLOGY, ELSEVIER, PHILADELPHIA, PA, vol. 127, no. 5, 1 November 2004 (2004-11-01), pages 1356-1371, XP005313112, ISSN: 0016-5085, DOI: 10.1053/J.GASTRO.2004.08.003
- CHEN ET AL: "Pre-S Deletion and Complex Mutations of Hepatitis B Virus Related to Advanced Liver Disease in HBeAg-Negative Patients", GASTROENTEROLOGY, ELSEVIER, PHILADELPHIA, PA, vol. 133, no. 5, 1 November 2007 (2007-11-01), pages 1466-1474, XP022407054, ISSN: 0016-5085, DOI: 10.1053/J.GASTRO.2007.09.002
- CHIEN-HUNG CHEN ET AL: "Combined Mutations in Pre-S/Surface and Core Promoter/Precore Regions of Hepatitis B Virus Increase the Risk of Hepatocellular Carcinoma: A Case-Control Study", THE JOURNAL OF INFECTIOUS DISEASES, vol. 198, no. 11, 1 December 2008 (2008-12-01), pages 1634-1642, XP055194702, ISSN: 0022-1899, DOI: 10.1086/592990
- NAAZNEEN MOOLLA ET AL: "Regulatory elements of hepatitis B virus transcription", JOURNAL OF VIRAL HEPATITIS, vol. 9, no. 5, 1 September 2002 (2002-09-01), pages 323-331, XP055194699, ISSN: 1352-0504, DOI: 10.1046/j.1365-2893.2002.00381.x
- Aleem Siddiqui ET AL: "Transcriptional control elements of hepatitis B surface antigen gene", , 1 February 1986 (1986-02-01), pages 566-570, XP055194416, Retrieved from the Internet: URL:http://www.pnas.org/content/83/3/566.f ull.pdf [retrieved on 2015-06-09]
- SHAMAY, M. ET AL.: 'HBV integrants of hepatocellular carcinoma cell lines contain an active enhancer' ONCOGENE vol. 20, 18 October 2001, pages 6811 - 6819, XP055155255
- QIN, J. ET AL.: 'Prospero-related homeobox protein(Proxl) inhibits epatitis B virus replication through repressing multiple cis regulatory elements' JOURNAL OF GENERAL VIROLOGY vol. 90, 04 March 2009, pages 1246 - 1255, XP055155258
- LIU, H. ET AL.: 'In vitro transfection of the hepatitis B virus PreS2 gene into the human hepatocarcinoma cell line HepG2 induces upregulation of human telomerase reverse transcriptase' BBRC vol. 355, 06 February 2007, pages 379 - 384, XP005899660

## Description

### BACKGROUND OF INVENTION

### Field of the Invention

The present disclosure generally relates to a cis acting element which enhances translation of proteins in eukaryotic cells.

### Description of the Related Art

Genes are expressed into proteins through transcriptional and translational processes in cells, which are subject to a variety of regulations. Among them are the use of a specific regulatory sequence such as a promoter, enhancer, Kozak sequence, and 3' untranslated region and the like. The regulatory sequences are acting usually located at the 5' upstream of or the 3' downstream of a gene and interacting with a variety of transcription or translation factors in the process.

In recent years, proteins have become widely used as therapeutic agents and thus protein expressions particularly the expression of proteins in eukaryotic cells in high efficiency has become an important issue. Many proteins are known to undergo post translational modification, which often determines the functional activities thereof. For this reason, eukaryotic cells are preferred as protein production cells compared to prokaryotic cells.

At present, various commercial vectors for protein expression in eukaryotic cells such as pGL4.10[luc2], pTargeT™, and pCI-neo and the like have been used. These vectors include in them various promoters and/or enhancers derived from a variety of species and genes.

However, for protein expressions in cell-free systems or eukaryotic cells from various origins and characteristics, there still exist needs for the development of regulatory sequences that can enhance the exogenous protein expression in eukaryotic cells.

KR 100758727 (B1) relates to a composition for diagnosis of the progress of liver disease of a HBV (Hepatitis B Virus) infected patient and a kit containing the same composition to anticipate, diagnose and prevent the progress of liver disease of the HBV infected patient by confirming nucleotide deletion of a preS1 initiation site.

KR 2002 0013372 (A) relates to hepatitis B virus vectors for the gene therapy, therefore a liver cell can be selectively treated by transferring a gene for the gene therapy using the vectors, wherein the circular hepatitis B virus plasmid vectors contain alpha-element having a specific nucleotide sequence and beta-element having a specific nucleotide sequence which are cis-acting elements necessary for the replication of HBV genome, in which the circular vectors contain an initial promoter of cytomegalo virus, DR1 element, epsilon element, alpha-element, DR2 element, beta-element and DR1 element.

### DETAILED DESCRIPTON OF THE INVENTION

### SUMMARY OF THE INVENTION

The present disclosure is to provide cis-acting regulatory elements which are able to effectively enhance the translational efficiency of proteins in eukaryotic cells.

The present inventors have identified that preS1 region from Hepatitis B virus containing particular mutations can function as a cis-acting regulatory element to enhance the expression of proteins under a promoter in eukaryotic cells.

In one aspect, the present disclosure provides cis-acting regulatory elements for eukaryotic cell expression, as defined in claims 1 to 3.

In another aspect, the present disclosure provides expression vectors for mammalian cell expression containing the cis-acting elements of the present disclosure, as defined in claims 4 to 8.

In a further aspect, the present disclosure provides mammalian cells transfected with the vector as described above, as defined in claims 9 and 10.

In a still further aspect, the present disclosure provides methods for producing and/or enhancing the translational level of recombinant proteins using the present cis-acting elements, as defined in claims 11 and 12.

### BRIEF DESCRIPTION OF THE DRAWINGS

These and/or other aspects and advantages of the invention will become apparent and more readily appreciated from the following description of the embodiments, taken in conjunction with the accompanying drawings of which:
In the following description, SEQ ID NO: 2, 4 to 6, 11 to 13, 19 and 20 relate to polynucleotides according to the invention. SEQ ID NO: 1, 3, 7 to 10, 14 to 18 and 21 relate to reference aspects.

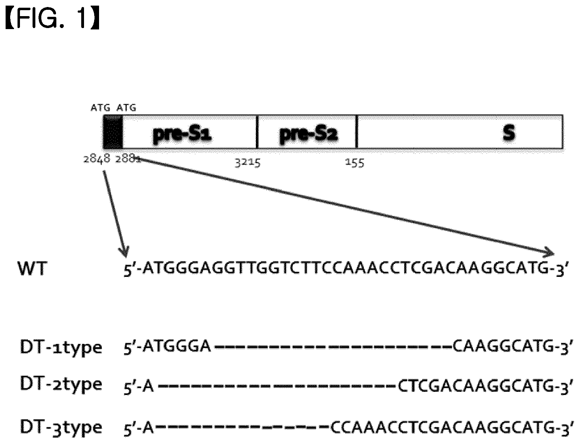

Fig. 1 is a schematic diagram showing the sequence of a wild type preS 1 region and the various deletion mutations in preS 1 start region found by the present inventors present specifically from a chronic hepatitis patient infected with Korean HCV genotype C type, which includes a type with 21 nucleotide deletion in the central region (DT-1 type), a type with 21 nucleotide deletion near the starting point (DT-2 type) and a type with 15 deletion near the starting point (DT-3 type). Due to the mutation, the start codon was affected in DT-2, DT-3) and thus codon 11 becomes the start codon (ATG), resulting in the creation of a novel cis-acting element (SEQ ID NO: 1, 2 and 3).

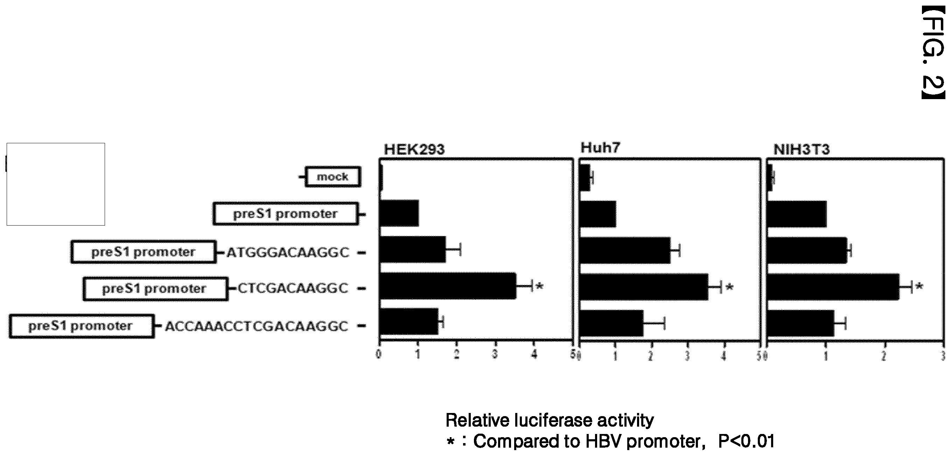

Fig 2 is a graph showing the effect of the specific nucleotide sequence derived from the deletion mutation in preS1 starting region found by the present inventors on the expression of a luciferase gene. Here each nucleotide sequence represented by SEQ ID NOs: 1, 2 and 3 was cloned into a recombinant expression vector pGL4.10 (Promega) with HBV preS1 promoter, each of which was then transfected into HEK293(human kidney embryonic kidney cell line), Huh7 (human hepatoma cell line) and NIH3T3(mouse embryonic fibroblast cell line), and the luciferase activities from each transfected cell line were measured. As shown in the figure, regardless of the cell types used, the cis-acting element of the present disclosure increased the level of protein expression.

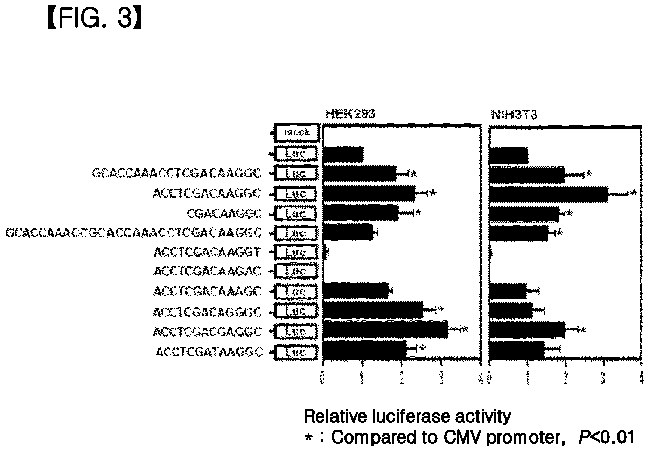

Fig. 3 is a graph showing the effect of the various specific nucleotide sequences derived from the deletion mutation in preS1 starting region (SEQ ID NOs: 4, 5, 6 and 7) and the nucleotide sequences with one substitution mutation at various positions of SEQ ID NO: 5 (SEQ ID NOs: 8, 9, 10, 11, 12, 13) on the expression of luciferase gene under the control of a CMV promoter. Here each of the sequences was cloned into a pIRES2-EGFP(enhanced green fluorescent protein) with a CMV promoter, each of which was then transfected into HEK293 and NIH3T3 cell lines and the luciferase activities from each transfected cell line were measured. Renilla plasmid was contransfected with the experimental vectors as control and its activity was also measured. As shown in the figure, the vectors containing cis-acting element of the present disclosure (SEQ ID NOs: 4, 5, 6 and 7) and the one with a substitution in the nucleotide sequence represented by SEQ ID NO; 5 (SEQ ID NOs: 10, 11, 12 and 13) increased the expression level of luciferase under the CMV promoter regardless of the types of cells used compared to the control. In contrast, cis-acting element having a substitutions at the first (C-> T) (SEQ ID NO: 8) and the second (G->A) (SEQ ID NO:9) from the 3' end has shown to repress the luciferase expression.

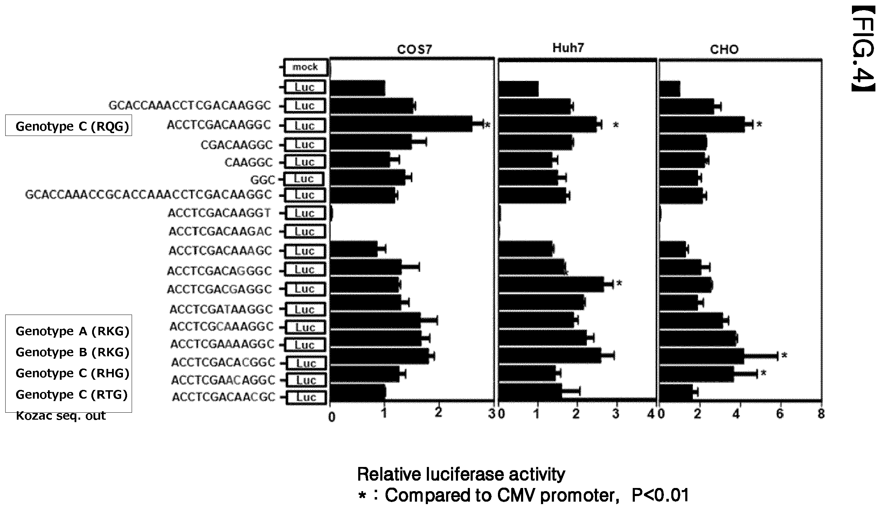

Fig. 4 is a graph showing the effect of the following sequences on the expression level of a luciferase gene: the nucleotide sequence derived from HBV having a particular genotype (Genotype A, B and C) different from the genotype where the preS1 deletion mutations were found; the nucleotide sequences with G to C substitution in Kozac sequence at the third nucleotide prior to ATG start codon (SEQ ID NOs: 4, 5, 6, 7, 8, 9, 10, 11, 12, 13, 15, 16, 17, 18, 19, 20, 21). Each sequence was cloned into a pIRES2-EGFP vector (Clontech) and transfected into COS7 (monkey kidney cell line), Huh7, CHO (Hamster ovary cell line) cell lines and the luciferase activities from each transfected cell line were measured. As a control, Renilla plasmid was contransfected with the experimental vectors and its activity was also measured. As shown in the figure, The vectors containing the cis-acting elements (SEQ ID NOs: 4, 5, 6, 7, 15, 16) and other tested elements from HBV of different genotypes (SEQ ID NOs: 17, 18, 19, 20) increased the expression level of luciferase under the CMV promoter regardless of the types of cells used compared to the control. In contrast, cis-acting element having substitutions at the first (C-> T) (SEQ ID NO: 8) and the second (G->A) (SEQ ID NO:9) nucleotide from the 3' end has shown to repress the luciferase expression. It also was found that the cis acting element with a substitution mutation at Kozac sequence (SEQ ID NO: 21) increased the luciferase expression. In FIG. 4, the nucleotides indicated in red are the nucleotide which was changed from that of SEQ ID NO: 13.

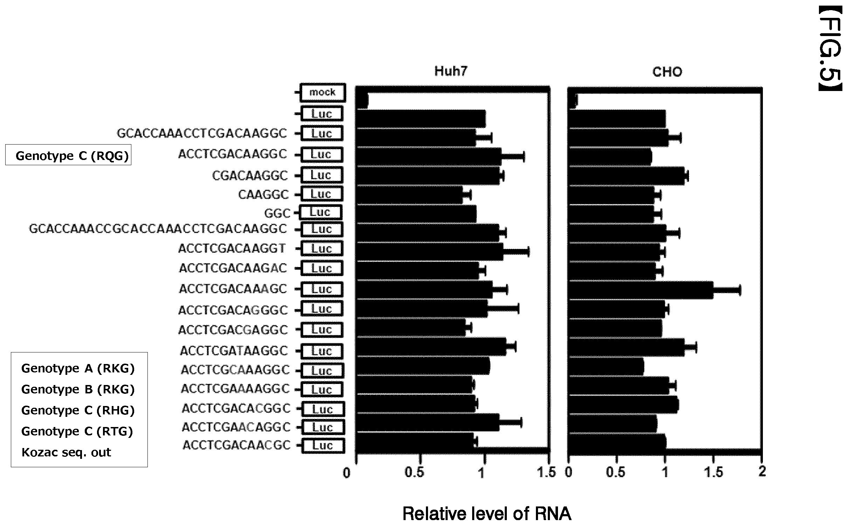

Fig. 5 is a result of quantitative real time PCR showing the effect of the present cis-acting elements on the expression level of luciferase at RNA level. Here the vectors as described in Fig. 4 were transfected into Huh and CHO (hamster ovary cell lines) cell lines, from which total RNA was extracted using a RNA extraction kit (Qiagen) and cDNA was synthesized therefrom. The cDNA synthesized was used for qPCR using the following primers: luci-RealF (SEQ ID NO: 22, 5'-TGC CCG CGA CCC CAT CT-3) and luci-RealR (SEQ ID NO: 23, 5'-CCC GAA AGC CGC AGA TCA AGT AGC-3'). As a control, the neomycin sequence in pIRES-EGFP was also amplified using the following primers: RE-NeoF (SEQ ID NO: 24, 5'-TGG AGA GGC TAT TCG GCT ATG-3') and RE-NeoR (SEQ ID NO: 25, 5'-TGC CTC GTC TTG CAG TTC ATT-3'). As shown in the figure, it was found that there was no changes in the expression of luciferase at the mRNA level in the presence of various cis-acting elements tested, which contains a nucleotide substitution at a site specific to HBV.

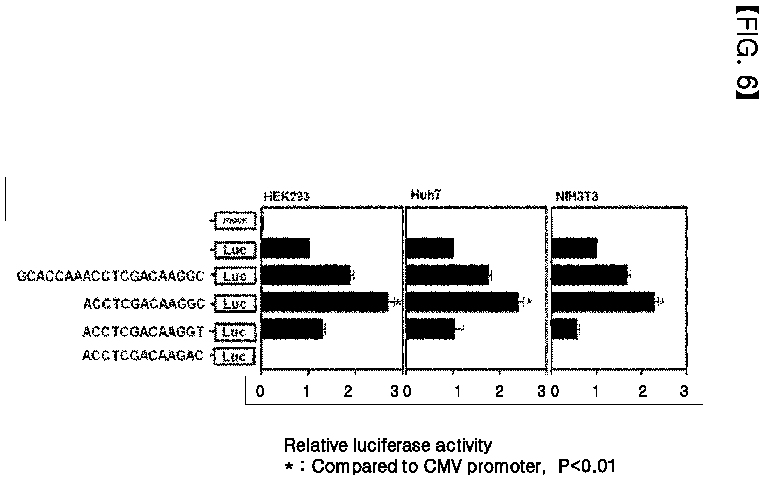

Fig. 6 is a graph showing effect of the various cis-acting elements as described in Fig. 3 on the expression level of luciferase under the control of CMV promoter contained in a different vector. Here the cis-acting element was cloned into a pcDNA3.1/CT-GFP fusion vector, which was transfected into Huh7 and CHO cell lines and the luciferase activity was measured. As a control, plasmid expressing Renilla waa cotransfected with the test plasmids and its activity was measured. As shown in Fig. 6, the present cis-acting elements (SEQ ID NOs: 4 and 5) increased the expression level of the protein tested regardless of the types of cell lines tested in comparison to the control. In contrast, it was found that luciferase activity was repressed in the cells transfected with vector containing the cis-acting elements with C→T mutation (SEQ ID NO: 8) and G→A mutation (SEQ ID NO: 9).

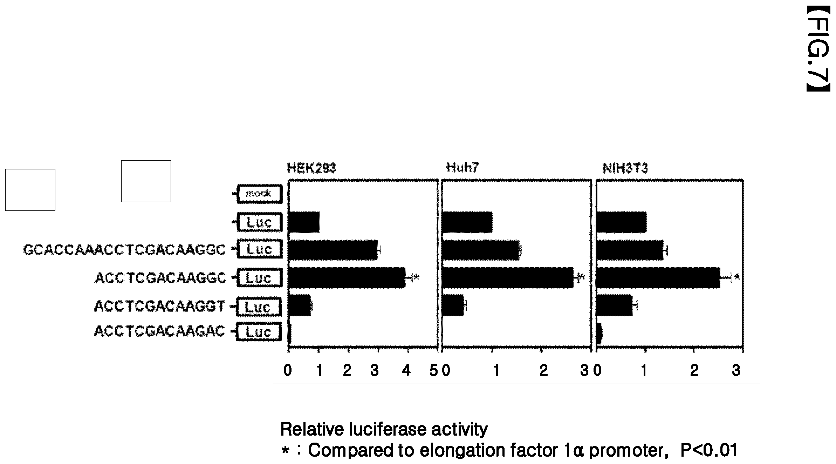

Fig. 7 is a graph showing the effect of the various cis-acting elements (SEQ ID NOs: 4, 5, 8 and 9) on the expression level of luciferase under the control of elongation factor 1α promoter. Each of the cis-acting elements was cloned into a GFP fusion TOPO vector (Invitrogen), which was then transfected into cell lines, HEK293, Huh7 and NIH3T3 and the luciferase activity from each transfected cells was measured. As shown in the figure, the cis-acting elements (SEQ ID NOs: 4 and 5) increased the protein expression level under the control of elongation factor 1α promoter regardless of the types of cells used in comparison to the control. In contrast, it was found that luciferase activity was repressed in the cells transfected with vector containing cis-acting elements with C→T mutation (SEQ ID NO: 8) and G→A mutation (SEQ ID NO: 9).

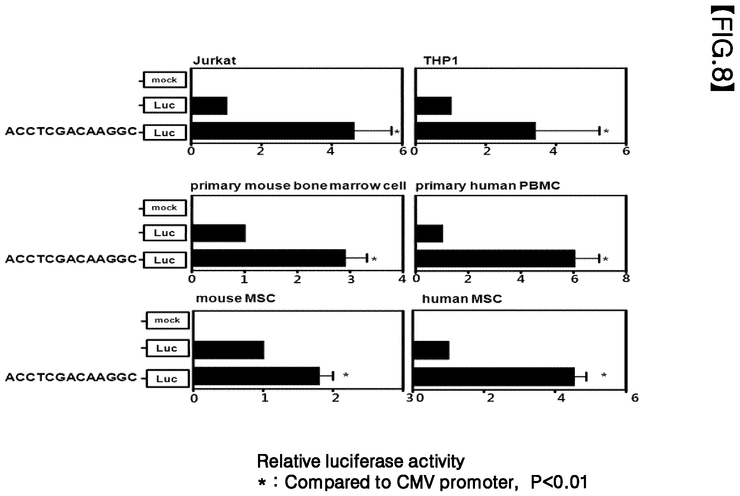

Fig. 8 is a graph showing the effect of cis-acting element represented by SEQ ID NO:5 on the luciferase expression in various cell lines from different species and origins. The pIRES2 vector containing luciferase linked to the prsent cis-acting element of SEQ ID NO: 5 was transfected into Jurkat (human T lymphocyte cell line), THP1 (human mononuclear cell line), primary mouse bone marrow cell, primary human peripheral blood mononuclear cell, mouse mesenchymal stem cell(MSC) and human MSC. As a result, it was found that the present cis-acting element increased the luciferase expression regardless of the cells types from various origins and species.

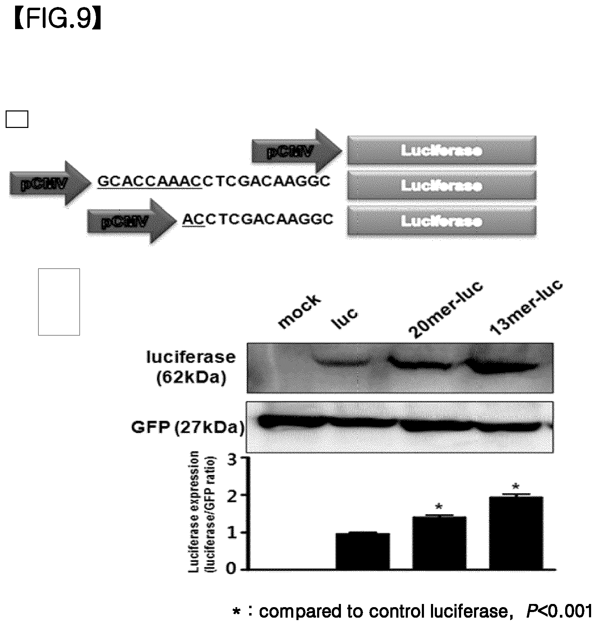

Fig. 9 is a result of the western blot showing the effect of the present cis-acting elements (SEQ ID NOs: 4 and 5) on the expression of luciferase gene under the control of a cytomegalovirus promoter. The cis-acting elements were cloned into a pIRES2-EGFP (Clontech) vector, which were then transfected into CHO cells, and the expression level of the luciferase was measured by Western blot using an antibody specific to the luciferase. As a result, the cis-acting element increased the expression level of luciferase compared to the luciferase without the cis-acting element in ovarian hamster cells.

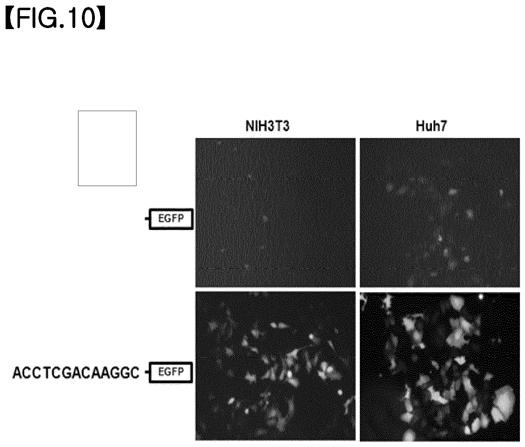

Fig.10 is a result showing the effect of cis-acting sequence represented by SEQ ID NO: 5 on the expression of EGFP under the control of cytomegalovirus promoter. For this, the ci-acting sequence was cloned into pcDNA3.1/CT-GFP fusion (Invitrogen) to link it to enhanced GFP. Then the vector was transfected into Huh7 and NIH3T3 cell lines and the cells expressing EGFP were examined using a fluorescent microscope. As shown in the figure, the expression of EGFP was markedly hanced by the present cis-acting element compared to the control where no cis-acting element was present.

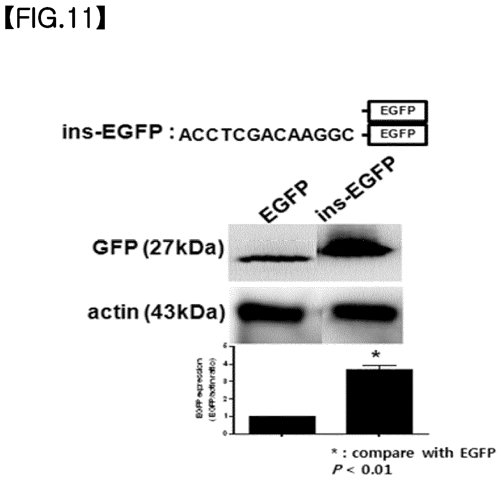

Fig. 11 is a result showing the effect of cis-acting sequence represented by SEQ ID NO: 5 on the expression of EGFP under the control of cytomegalovirus promoter. For this, the ci-acting sequence was cloned into pcDNA3.3-TOPO (Invitrogen) to link it to enhanced GFP. Then the vector was transfected into COS cell line. Then the cells expressing EGFP were examined by western blot using an antibody specific to GFP, where the beta actin was used as control. As shown in the figure, the expression of EGFP was enhanced by the present cis-acting element compared to the control where no cis-acting element was present in monkey kidney embryonic mesenchymal cell line.

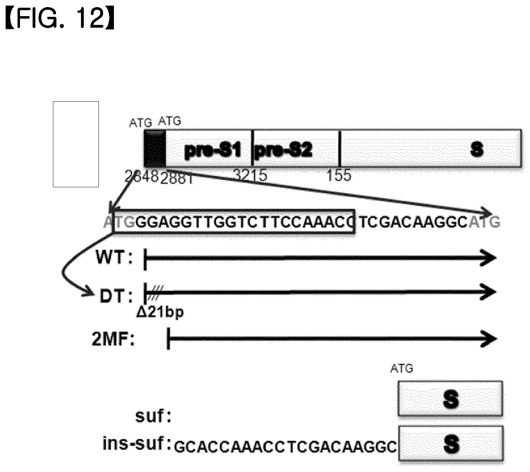

Fig. 12 is schematic diagrams of the recombinant expression vector pIRES2-EGFP (Clontech) and the gene inserted into the vector to examine the effect of the present cis-acting element on the expression of large surface antigen and small antigen under the control of cytomegalovirus promoter. WT represents a nucleotide sequence of a normal large surface antigen, DT represents a nucleotide sequence having 21 nt deletion from the 5' end, and 2MF is a nucleotide sequence constructed to express large surface antigen from ATG, 33 nt downstream from WT starting codon. Suf represents a small surface antigen, and in-surf represents the 20 mer nucleotide sequence of SEQ ID NO: 4 linked 5' to the small surface antigen. This shows that the present cis-acting elements enhances the expression of a surface antigen at the translational stage without affecting the transcription and thus can be utilized for the over expression of a protein.

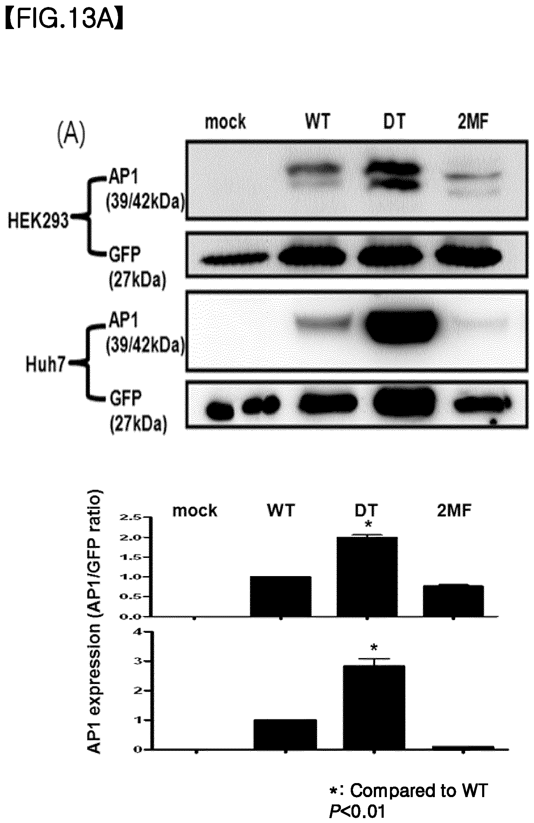

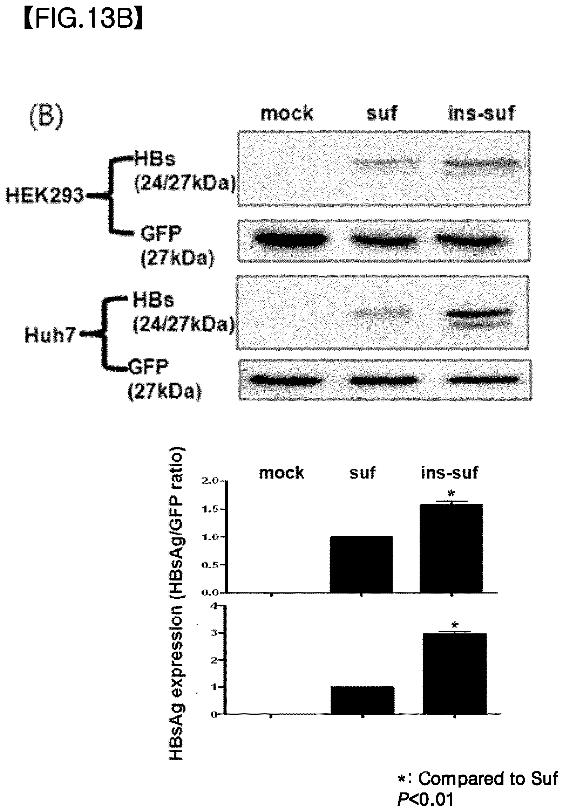

Fig. 13A and B are a western blot result using eukaryotic cells transfected with various recombinant vectors expressing large or small surface antigen constructed in Fig. 12. HEK293 and Huh7 cell lines were transfected with the corresponding vectors and the protein extracts from each cell were used for western blot analysis using antibody against large surface antigen API and antibody against small surface antigen HBs. An antibody against GFP was used as a control. As a result, it was found that the cis-acting element (SEQ ID NO: 14) enhanced the expression of large surface antigen, compared to the control. Also the cis-acting element (SEQ ID NO: 4) also enhanced the expression of small surface antigen.

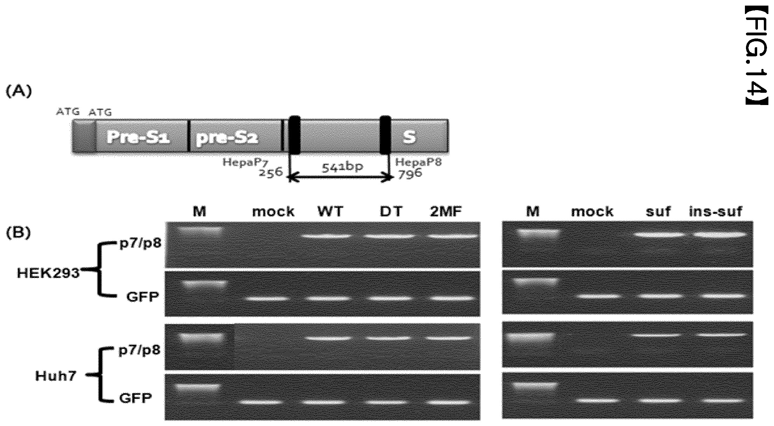

Fig. 14 is a result showing the effect of various recombinant vectors constructed in Fig. 13 on the expression HBV large surface antigen and small antigen at RNA level in eukaryotic cells. Part (A) shows positions of primers used to amplify a small surface antigen, a primer that can amplify GFP contained in pIRES2 used as a control and their sequences. Part (B) shows a RT-PCR result to measure RNA expression level. Here the plasmids were transfected into HEK293 and Huh7 cell lines, from which RNA was extracted using RNA extraction kit (Qiagen). Then the RNA was used for RT-PCR using the following primers which can specifically amplify a small surface antigen: HepaP7 (SEQ ID NO: 26 ; 5' - GTG GTG GAC TTC TCT CAA TTT TC - 3') and HepaP8 SEQ ID NO: 27; 5' - CGG TAW AAA GGG ACT CAM GAT - 3'). As a control, the following primers specifically amplifying GFPF were used:(SEQ ID NO: 28; 5'-ATG GTG AGC AAG GGC GAG GA-3') and GFPR(SEQ ID NO:29; 5'-TTA CTT GTA CAG CTC GTC CA-3'), As a result, it was found that expression of all the large surface antigen genes from wild type, mutational strain and strain with 33 nt deletion was not changed at the RNA level.

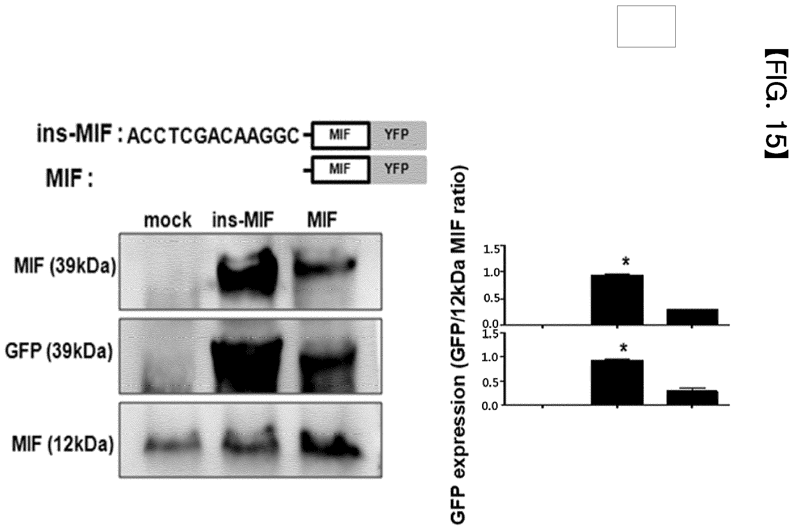

Fig. 15 is a result showing the effect of the cis-acting sequence represented by SEQ ID NO: 5 on the expression of MIF and GFP under the control of cytomegalovirus promoter. For this, wild type MIF gene or MIF-YFP gene were cloned into pEYFP-Nl (Clontech) vector to construct a fusion protein expression vector. To test whether the cis-acting element of SEQ ID NO:5 enhances the expression of MIF-YFP fusion protein, recombinant vector expressing MIF fused with enhanced GFP was sub-cloned into pcDNA3.3-TOPO vector. Such vectors were then transfected into Huh7 cells from which protein extracts were prepared and used for western blot analysis using antibodies specific to MIF and GFP. As a result, it was found that the cis-acting element (SEQ ID NO: 5) enhances the protein expression of MIF and YFP compared to the wild type large surface antigen.

### DETAILED DESCRIPTION OF THE EMBODIMENTS

In one embodiment, the present invention provides cis-acting elements for eukaryotic expression selected from the group consisting of polynucleotide represented by SEQ ID NOs: 2, 4, 5, 6, 11, 12, 13, 19 and 20. SEQ ID NO: 1, 3, 7 to 10, 14 to 18 and 21 relate to reference aspects.

The present inventors endeavored to develop a cis acting regulatory element which is able to effectively enhance the translational efficiency of proteins in eukaryotic cells. As a result, it have been found that preS1 starting region from Hepatitis B virus containing particular mutations can function as a cis-acting element to markedly enhance the expression of proteins under a promoter in eukaryotic cells.

It has been known that 30 million people worldwide have been infected with Hepatitis B Virus (HBV), which eventually leads to an acute or chronic hepatitis, cirrhosis or hepatoma and the like, resulting in the increase of morbidity rate. HBV infection shows a variety of clinical course such as infection with no symptoms, chronic hepatitis, cirrhosis or hepatoma, which are thought to be resulted from sequence mutations and/or genotypes found in HBV. According to recent findings, it has been reported that majority of genotype found in Korea is genotype C (Kim BJ et al., Korean J. Gastroenterol, 42(6):496-501(2003)). Most of HBV is found to belong to genotypes A, B or C. The C type is more virulent than the other types and each type is found almost at the same level. In contrast, in Korea, HBV found includes genotype C at a higher ratio than the other two types.

The majority of phospholipids present in HBV envelope are originated from the envelope of host cells. In contrast the surface antigens on the envelope are derived from HBV. The surface antigens are a protein complex consisting of 3 types of proteins named as Large (L), Medium (M) and Small (S) according to their sizes. Each of the three types of proteins is synthesized from one Open Reading Frame (ORF) which is transcribed from one gene called *env.* Namely, *env* gene contains three ATG start codons starting from the 5' upstream of the gene and is known to encode three proteins: LHB (large hepatitis B surface antigen) that is 400 amino acids long translated from the first ATG to the stop codon; MHB (medium hepatitis B surface antigen) that is translated from the second ATG which is located 119 amino acids downstream of the first ATG to the stop codon that is common to all three proteins; and SHB (small hepatitis B surface antigen) that is translated from the third ATG to the stop codon.

SHB protein of 226 amino acids is known as S-domain and 55 amino acids upstream of the S-domain at the N-terminal which is only present MHB is called preS2, and preS2+S, namely 119 amino acids upstream of the MHB at the N-terminal is called preS1. Depending on the subtypes of HBV, preS1 is either consisting of 108 amino acids (ay-type) or 119 amino acids (ad-type), which is more universal. The second amino acid, glycine, of preS1 is myristoylated, through which the end of the preS1 interacts with the membrane and defines the entire structure of HBsAg. The preS1 and preS2 do not have a transmembrane region and in contrast S-domain has a total of 3 (or 4) hydrophobic transmembrane region. Proteins with a transmembrane region usually have a fixed topology and thus their function is determined according to the direction each of them takes in relation to the membrane, inward or outward of the membrane. In case of LHB, 174 amino acids of pre S which corresponds to half of the protein faces outside of the membrane, and the other half of preS faces inside of the membrane.

The preS1 includes a region through which HBV binds to liver cells and S antigen contains S promoter which regulates the transcription level. Thus the genetic mutation in this region influences on the expression of S antigen. The preS 1 includes a region that is a target in host cells at B and T cell level, and thus is known to be associated with acute hepatitis and hepatoma. Among HBV variants, the genetic mutations in preS1 is known to be associated with the development of hepatoma. The mechanism by which the genetic mutation causes the hepatoma is not clear ((Chisari FV et al., Annu Rev Immunol. 13:29-60(1995).

The messenger RNA is known to contain a region called kozak consensus sequence to which a ribosome binds during the translation process, which includes ACCAUGG, and is a key factor in determining the translational efficiency with ATG start codon (MarilynKozak, PNAS, 87;8301-8305(1990)). Since it was first identified in 1987 from a mRNA of 699 nts (Marilyn Kozak, Nucl. Acid. Res.,15(20):8125-8148(1987)), more genes with the Kozak sequence have been identified and various polymorphisms were identified to be present and it remains a controversial whether the substitution mutations in the Kozak sequence lead to the changes in the translational efficiencies.

The present cis-acting element was found to increase the translational efficiency in eukaryotic cells.

As evident from the present disclosure including examples, the present cis acting elements derived with mutations derived from HBV preS1 starting region increased the translation of a target gene compared to the wild type control in animal cells. Also it increased the protein expression in the vector systems having a variety of promoters and the cells of various origins. For example, when HBV preS1 promoter and the present cis acting element (SEQ ID NO:2) were transfected into HEK293, Huh7 and NIH3T3 and its effect on the protein expression was examined by luciferase activity, compared to the control in which only the preS1 promoter was used, the cells transfected together with the present cist acting element have been found to increase the luciferase activity by about 3.3 times in HEK293 cells, by about 3.2 times in Huh7 cells and by about 2.1 times in NIH3T3 cells (refer to FIG. 2). The vector that contained the present cis acting element has been found to increase the protein expression regardless of the cells it was transfected compared to the control. That is, this indicates the excellent effect of the present cis acting element on the protein expression level compared to the wild type preS1.

The present cis-acting element increase the protein expression of HBV, particularly the protein expression of the surface antigen of HBV.

As demonstrated in the examples described herein below, when the sequence of the wild type preS1 starting region linked to the gene encoding HBV large surface antigen or the sequence of the preS1 starting region with mutation (SEQ ID NO: 14) linked to the gene encoding HBV large surface antigen was transfected into each of HEK293 and Huh7 and their effects on the protein translation were examined by western blot, it showed that the sequence of the preS1 starting region with mutation (SEQ ID NO: 14) linked to HBV large surface antigen gene increased the protein expression by about 3.3 times in HEK293 cells, and by about 3.2 times in Huh7 cells and by about 2.1 times in NIH3T3 cells measured by the luciferase activity (refer to FIG. 13).

In other aspect of the present disclosure, the present invention provides a vector for expression in animal cells comprising a cis-acting element selected from the group consisting of polynucleotide represented by SEQ ID NOs: 2, 4, 5, 6, 11, 12 and 13, and a promoter operable in eukaryotic cells.

The vector of the present disclosure markedly increases the efficiency of protein expression.

In the present vector, the cis-acting element may be located upstream or downstream of the nucleotide sequence coding for a protein of interest, preferably upstream of the gene of interest.

In one preferred embodiment of the present disclosure, the present vector for expression in animal cells increases the expression of protein of interest compared to the vector that contains the wild type preS1 starting region and a promoter for expression in animal cells. For example, HBV preS1 promoter with the present cis-acting element (SEQ ID NO:2) was transfected into each of HEK293, Huh7 and NIH3T3 cells and its effect on the protein expression was measured by a luciferase activity. As a result, the vector comprising the present cis acting element increased the protein expression by about 3.3 times in HEK293 cells, and by about 3.2 times in Huh7 cells and by about 2.1 times in NIH3T3 cells measured by the luciferase activity (refer to FIG. 2). This indicates the excellent effect of the present vector comprising the cis-acting element derived from HBV preS1 starting region on the protein expression level compared to the wild type preS 1.

In one preferred embodiment of the present disclosure, the present vector further comprises a nucleotide sequence encoding a protein of interest, particularly virus antigens, fluorescent proteins, hormons, cytokines, antibodies, peptides, aptamers, AdNectin, affibody (US Patent No: 5,831,012), Avimer (Silverman, J. et al, Nature Biotechnology 23(12):1556(2005)) or Kunitz domain (Arnoux B et al., Acta Crystallogr. D Biol. Crystallogr.58(Pt 7):12524(2002); Nixon, AE, Current opinion in drug discovery & development 9(2):2618(2006)). More particularly, the proteins which may be expressed by the present vector are viral antigens or fluorescent proteins. Most particularly, they includes HBV surface antigens , GFP (green fluorescent protein) or YFP (yellow fluorescent protein).

As demonstrated in the examples described herein below, the cis-acting element (SEQ ID NO:5) and MI were cloned into a vector having YFP and the resulting vector was transfected into Huh7 cells, which was then analyzed by western blot. As a result, the expression of MIF was increased by 2.9 times compared to the wild type MIF-YFP vector, and the YFP expression was increased by more than 3 times (refer to FIG. 15).

According to one preferred embodiment, the promoter operable in animal cells includes a HBV promoter, a CMV(Cytomegalovirus) promoter, an Adenovirus late promoter, a Vaccinia virus 7.5K promoter, a SV40(Simian Virus 40) promoter, SV40E1 promoter, a tk promoter of HSV(Herpes simplex virus), a RSV(Respiratory syncytial virus) promoter, an EF1 (elongation factor-1 alpha) promoter, a metallothionein promoter, a beta actin promoter, a promoter of human IL-2(interleukin-2) gene, a promoter of human IFN(interferon) gene, a promoter of human IL-4(interleukin-4) gene, a promoter of human lymphotoxin gene or a promoter of human GM-CSF(Granulocyte macrophage colony-stimulating factor), more particularly a CMV promoter, an EF1 alpha promoter, a metallothionein promoter or beta actin promoter, most particularly a CMV promoter.

As demonstrated in the examples described herein below, the animal expression vector pIRES2-EGFP comprising the present cis-acting element of the present disclosure (SEQ ID Nos: 5 or 12) and the CMV promoter was transfected into each of HEK293, Huh7 and NIH3T3 cells and their effects on the protein translation were examined by measuring the luciferase activity. As a result, the sequence represented by SEQ ID NO: 5 has increased the luciferase activity by about 2.4 times in HEK293 cells, and by about 3.8 times in Huh7 cells and by about 3.1 times in NIH3T3, and the sequence represented by SEQ ID NO: 12 has increased the luciferase activity by about 3.1 times in HEK293 cells, and by about 3.1 times in Huh7 cells and by about 1.9 times in NIH3T3 (Refer to FIG.3).

Also various expression vectors and promoter when used with the present cis-acting elements showed the similar results when tested for the protein expression.

The animal expression vector pcDNA3.1/CT-GFPfusion comprising the present cis-acting element of the present disclosure (SEQ ID No: 5) and the CMV promoter was transfected into each of HEK293, Huh7 and NIH3T3 cells and their effects on the protein translation were examined by measuring the luciferase activity. As a result, it has increased the luciferase activity by about 2.7 times in HEK293 cells, and by about 2.4 times in Huh7 cells and by about 2.2 times in NIH3T3 (Refer to FIG. 5).

The animal expression vector comprising the present cis-acting element of the present disclosure (SEQ ID No: 5) and the EF1 α promoter was transfected into each of HEK293, Huh7 and NIH3T3 cells and their effects on the protein translation were examined by measuring the luciferase activity. As a result, it has increased the luciferase activity by about 3.8 times in HEK293 cells, and by about 2.7 times in Huh7 cells and by about 2.5 times in NIH3T3 (Refer to FIG. 6).

According to one preferred embodiment of the present disclosure, the sequence represented by SEQ ID NO: 5 increased the protein expression at the highest level compared to other cis-acting element of the present disclosure with various promoters (preS1 promoter, CMV promoter and EF1 a promoter).

In one embodiment, the cis-acting element of the present disclosure is represented by SEQ ID NO: 5. Also encompassed by the sequence represented by SEQ ID NO: 5 are sequences that are substituted with other nucleotide at the 3' and/or 5' ends. Also as long as the protein expression is increased, also encompassed by the sequence represented by SEQ ID NO: 5 is sequences to which some nucleotide sequences are added at the 3' and/or 5' ends.

According to one preferred embodiment of the present disclosure, the present vectors are used for protein expression in animal cell. The animal cells includes cells derived from mammals, rodents, birds or insects, more particularly CHO(Chinese hamster ovary) cells,HEK293(human embryonic kidney) cells, Huh7 cells, NIH3T3 cells, VERO cells, HeLa cells, WI38 cells, BHK(Baby hamster kidney) cells, COS cells or MDCK(Madin-Darby Canine Kidney) cells, more particularly, HEK293 cells, Huh7 cells, NIH3T3 cells or COS7 cells, most particularly, HEK293 cells.

In other aspect the present disclosure provides a cell transformed with the present vector.

In a further aspect the present disclosure provides a method for producing a recombinant proteins comprising a step of culturing the cells transformed with the present vector.

In a further aspect the present disclosure provides a method for expressing a protein in eukaryotic cells, the method comprising a step of introducing a vector which comprises the cis-acting element of the present disclosure and a promoter and an exogenous gene operably linked to the gene and expressing the protein of interest from the exogenous gene. The exogenous genes are not found to be present as associated with the present cis-acting regulatory sequence and the promoter as described herein, and include nucleotide sequences encoding, for example, viral antigens, fluorescent proteins, hormones, cytokines, antibodies, peptide aptamers, AdNectin, affibody, Avimer or or Kunitz domain and RNA. The term operably linked means that nucleotide sequences are linked such a way that transcription followed by translation is normally initiated under the control of the present cis-acting regulatory sequence and promoter of interest.

The present cis-acting elements derived from HBV with various deletion are the one that has never been used or disclosed before the present invention to enhance the protein expression in the context of a protein expression vector in animal cells. It is evident from the following examples that the cis-acting element derived from HBV with a deletion mutation, when introduced into a vector, can increase the protein expression of the protein of interest in animal cells. For the production of a recombinant protein, the present cis-acting element is introduced into an appropriate expression vector and can be used to develop a method to increase the translational efficiency of the protein of interest thus increasing the productivity of the industrial production of the recombinant protein..

There are no reports at all about using the present cis-acting element to increase the productivity of a recombinant protein after the element being introduced into a vector.

For preparing the animal cells transformed with the present vector, the vector can be introduced into cells various methods known in the art. For example, the vectors can be introduced into the cells using methods such as microinjection (Capecchi, M.R., Cell, 22:479(1980)), calcium phosphate precipitation (Graham, F.L. et al., Virology, 52:456(1973)), electroporation (Neumann, E. et al., EMBO J., 1:841(1982)), liposome mediated transduction (Wong, T.K. et al., Gene,10:87(1980)), DEAE-dextran treatment (Gopal, Mol. Cell Biol., 5:1188-1190(1985)), and gene bombardment (Yang et al., Proc. Natl. Acad. Sci.,87:9568-9572(1990)) and the like.

The transformed animal cells of the present disclosure may be cultured various methods known in the art. The media which may be used for the culture includes for example, Eagles's MEM (Eagle's minimum essential medium, Eagle, H. Science 130:432(1959)), α-MEM (Stanner, C.P. et al., Nat. New Biol. 230:52(1971)), Iscove's MEM (Iscove, N.et al., J. Exp. Med. 147:923(1978)), 199 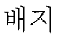 (Morgan et al., Proc. Soc. Exp. Bio. Med., 73:1(1950)), CMRL 1066, RPMI 1640 (Moore et al., J. Amer. Med. Assoc. 199:519(1967)), F12 (Ham, Proc. Natl. Acad. Sci. USA 53:288(1965)), F10 (Ham, R.G. Exp. Cell Res. 29:515(1963)), DMEM (Dulbecco's modification of Eagle's medium, Dulbecco, R. et al., Virology 8:396(1959)), mixture of DMEM and F12(Barnes, D. et al., Anal. Biochem. 102:255(1980)), Way-mouth's MB752/1 (Waymouth, C. J. Natl. Cancer Inst. 22:1003(1959)), McCoy's 5A (McCoy, T.A.,et al., Proc. Soc. Exp. Biol. Med. 100:115(1959)) and MCDB series (Ham, R.G.et al., In Vitro 14:11(1978)) and the like. The detailed description regarding the cell culture and media used therefor may be found for example in R. Ian Freshney, Culture of Animal Cells, A Manual of Basic Technique, Alan R. Liss, Inc., New York, which is incorporated herein by reference.

The present disclosure is based on the discovery of a new HBV variant having a deletion mutation at preS1 starting region. Thus in a reference aspect, the present disclosure provides a method to provide information necessary for diagnosing liver disease related to HBV infection by detecting the deletion type in preS1 region, DT-1 type, DT-2 type, and/or DT-3 type.

In another reference aspect, the present disclosure provides a method for diagnosing liver disease related to HBV infection by detecting the deletion type in preS1 region, DT-1 type, DT-2 type, and/or DT-3 type.

The disease related to HBV infection includes chronic liver disease, liver cirrhosis and liver cancer.

In the present methods, for detecting the deletion type in preS1 region, methods known in the art, for example, southern blot, DNA chip, PCR (polymerase chain reaction) and the like may be used. In one embodiment, PCR, particularly nested PCR method in which a second PCR is performed on a template which is the product of a first PCR. In one embodiment, PCR is performed using a set of forward and reverse primers represented by SEQ ID Nos: 30 and 31; or a set of forward and reverse primers represented by SEQ ID Nos: 32 and 33 without being limited thereto.

The present disclosure is further explained in more detail with reference to the following examples. These examples, however, should not be interpreted as limiting the scope of the present invention in any manner.

### EXAMPLES

### Materials and Methods

### Construction of vectors expressing luciferase under various of promoters

### Preparing HBV preS1 promoter and nucleotide sequence resulted from the deletion mutation

Prior informed consent was obtained from Patients visited Seoul National University hospital with HBV infection and the patient were randomly selected and tested for the presence of the deletion using nested PCR and direct sequencing. DNA extracted from patient having a deletion mutation and a wild type HBV was used as a template and then PCR was performed to isolate the deletion mutation and wild type preS1 promoter sequences of large surface antigen gene as in FIG. 1. The primers used comprise SacI and XhoI site at each end and the primer sequences are as follows: Forward Primer: Sac ProF(5'-GAG CTC CGC AGA AGA TCT CAA TCT CGG-3'), Reverse Primer Xho Nor ProR(5'-CTC GAG GCT GTA GCT CTT GTT CCC AA-3'), Xho Dell ProR(5'-CTC GAG GCC TTG TCC CAT TGC TGT AGC-3'), Xho Del2 ProR(5'-CTC GAG GCC TTG TCG AGT GCT GTA GC-3'), Xho Del3 ProR(5'-CTC GAG GCC TTG TCG AGG TTT GGT TGC TGT AGC-3').

### Construction of the vector expressing luciferase

To test the effect of the HBV promoter, 10µg of pGL4.10 vector (Promega, USA) expressing luciferase was digested with 10 U of Xho I and SacI and incubated for 18hours at 37°C followed by treatment with alkaline phosphatase (TAKARA BIO INC., Japan) according to the manufacturer's instruction. Then the vector and the PCR products prepared as described above were purified by PCRquick-spin™ PCR product purification kit (iNtRON Biotechnology INC, Korea) according to the manufacturer's instruction and they are ligated using T4 DNA ligase (TAKARA BIO INC., Japan) in which the ratio of the insert vs the vector was 3:1. The ligation was performed using 350 Unit of the ligase in the buffer 1 mM Tris-HCl pH 7.5, 5 mM KCl, 1 mM 2-mercaptoethanol, 0.01 mM EDTA, 5% Glycerol in a total volume of 10µl and incubated for 1 hour at 16°C. Then the ligation product was transformed into competent Top10 (Invitrogen Co., USA) cells for 30sec at 42°C and spreaded onto a LB agar plate containing ampicillin ((SIGMA-ALDRICH, INC, USA). Each of the colonies formed then was cultured in a agar broth containing ampicillin for 18hours, after which plasmid was extracted and purified using DNA-spin™ MIDI plasmid DNA purification kit (iNtRON Biotechnology INC Korea) from each culture in a total of 200µl.

### The construction of a luciferase gene linked to the present nucleotide sequence from the deletion mutation.

To construct a luciferase gene linked to a variety of the present nucleotide sequence from the deletion mutant, first luciferase gene was isolated using a pGL4.10 as a template and the primers as follows: Forward primer: LuciF(5'-ATG GAA GAT GCC AAA AAC ATT-3'), inlucF(5'-GCA CCA AAC CTC GAC AAG GCA TGG AAG ATG CCA AAA ACA TT-3'), Del1-insF(5'-ACC TCG ACA AGG CAT GGA AGA TGC CAA-3'), Del2-insF(5'-CGA CAA GGC ATG GAA GAT GCC AAA-3'), VDREF(5'-GCA CCA AAC CGC ACC AAA CCT CGA CAA GGC-3'), Del1-1F(5'-ACC TCG ACA AGG TAT GGA AGA TGC CAA A-3'), Del1-2F(5'-ACC TCG ACA AGA CAT GGA AGA TGC CAA A-3'), Dell-3F(5'-ACC TCG ACA AAG CAT GGA AGA TGC CAA A-3'), Del1-4F(5'-ACC TCG ACA GGG CAT GGA AGA TGC CAA A-3'), Dell-5F (5'-ACC TCG ACG AGG CAT GGA AGA TGC CAA A-3'), Del1-6F(5'-ACC TCG ATA AGG CAT GGA AGA TGC CAA A-3'), Reverse primer: LuciR(5'-TTA CAC GGC GAT CTT GCC GC-3'). The PCR products were then purified using an agarose gel extraction kit.

Also the present nucleotide sequences from the deletion mutant were cloned into a pIRES2 vector. Using a 13mer nucleotide sequence as a starting point, sequences found in other types of HBV (A, B and C type) and G to C substitution at the third base upstream of ATG codon in Kozac sequence linked to a luciferase gene were constructed using a site mutagenesis using the following primers: A-RKG-Luc2-F (5' - ACC TCG CAA AGG CAT GGA AGA TGC CAA AAA CAT TAA GAA G-3'), A-RKG-Luc2-R (5' -CTT CTT AAT GTT TTT GGC ATC TTC CAT GCC TTT GCG AGG T-3'), B-RKG-Luc2-F (5'- ACC TCG AAA AGG CAT GGA AGA TGC CAA AAA CAT TAA GAA G-3'), B-RKG-Luc2-R (5'-CTT CTT AAT GTT TTT GGC ATC TTC CAT GCC TTT TCG AGG T-3'), C-RHG-Luc2-F (5'-ACC TCG ACA CGG CAT GGA AGA TGC CAA AAA CAT TAA GAA G-3'), C-RHG-Luc2-R (5 '- CTT CTT AAT GTT TTT GGC ATC TTC CAT GCC GTG TCG AGG T-3'), C-RTG-Luc2-F (5'-ACC TCG AAC AGG CAT GGA AGA TGC CAA AAA CAT TAA GAA G-3'), C-RTG-Luc2-R (5'-CTT CTT AAT GTT TTT GGC ATC TTC CAT GCC TGT TCG AGG T-3'), Kozac-null-F (5'-AGC TTC GAA TTC GCC CTT GCC GCC ACC ATG GAA GAT GCC A-3'), Kozac-null-R (5'-TGG CAT CTT CCA TGG TGG CGG CAA GGG CGA ATT CGA AGC T-3').

### Construction of the vector expressing luciferase under the control of various promoters

Cloning was performed using TOPO TA cloning kit (Invitrogen Co) using the manufacturer's instruction. Plasmids were extracted and purified using DNA-spin plasmid DNA purification kit (iNtRON Biotechnology INC.) from 3ml of bacterial culture of 18 hrs. Fifty µl of the plasmid was treated with EcoRI and the fragment was purified using QIAEX II System kit (QIAGEN Inc., Germany).

Ten µg of the plasmid having CMV (cytomegalovirus) promoter pIRES2-EGFP(enhanced green fluorescent protein) (Clontech Laboratories INC., USA) was digested with 10U of EcoRI and incubated for 18hrs at 37°C followed by treatment with alkaline phosphatase (TAKARA BIO INC.) according to the manufacturer's instruction, which was then purified using PCRquickspin™ PCR product purification kit (iNtRON Biotechnology INC.). The purified pIRES2-EGFP vector and the PCR product as described above were ligated using T4 DNA ligase (TAKARA BIO INC) as described above. Also the present nucleotide sequence was cloned into the vectors namely GFP fusion TOPO vector having CMV promoter and pEF6/V5-HIS TOPO vector having human elongation factor 1 α promoter using TOPO TA cloning kit (Invitrogen Co) according to the manufacturer's instruction. After that the vectors as described above were transfected to competent cells Top 10 (Invitrogen Co) for 30sec at 42°C and the cells were spreaded on to a LB agar plate with appropriate antibiotic resistance gene namely pIRES2-EGFP vectors on the plate with Kanamycin; GFP-fusion TOPO vector and pEF6/V5-HIS TOPO on the plate with Ampicillin (SIGMA-ALDRICH, INC). The colonies formed were inoculated and cultured in 50ml of LB broth for 18 hrs and the plasmids therein were extracted using DNA-spin™ MIDI plasmid DNA purification kit (iNtRON Biotechnology INC.) in a total volume of 200µl.

### Construction of the present nucleotide sequence linked to EGFP

The pIRES2-EGFP vector comprising EGFP was used as a template for PCR to obtain a construct wherein EGFP was linked to the present nucleotide sequence using the primers as follows: Forward primer EGFPF(5'-ATG GTG AGC AAG GGC GAG GA-3'), ins-EGFPF(5'-ACC TCG ACA AGG CAT GGT GAG CAA GGG CGA GGA-3'), Reverse primer EGFPR(5'-TTA CTT GTA CAG CTC GTC CA-3'). The PCR products were purified using a DNA extraction kit from agarose gel.

### Construction of EGFP expression vector under the control of CMV promoter

The expression vectors GFP Fusion TOPO and pcDNA3.3-TOPO having CMV promoter and the GPF expression vectors prepared as described above were transfected to competent cells Top10 (Invitrogen Co) and DNA was extracted therefrom as described above.

### Construction of the present nucleotide sequence linked to a large surface antigen gene or a small surface antigen gene

The large surface antigen comprising HBV preS1 deletion, the large surface antigen comprising wild-type PreS1, and the large surface antigen having an ATG start codon located at 33 nt downstream from the 5' end of the large surface antigen were PCR amplified using HBV virus DNA as a template which was extracted from a patient infected with HBV preS1 deletion mutant strain and wild type strain as template using the following primers: Forward primer: DelF2(5'-GGG TCA CCA TAT TCT TGG G-3'), 2MF(5'-ATG GGG ACG AAT CTT TCT GTT-3'), Reverse primer: HB2R(5'-CAT ACT TTC CAA TCA ATA GG-3'). The PCR products were purified using a DNA extraction kit from agarose gel. Also the small surface antigen comprising the present deletion mutant nucleotide sequence was prepared by PCR using the following primers: Forward primer: Nor sufF(5'-ATG GAG AAC ACA ACA TCA GGA-3'), ins sufF(5'-GCA CCA AAC CTC GAC AAG GCA TGG AGA RCA CMA CAT CAG GAT TC-3'), Reverse Primer: Nor sufR(5'-TCA AAT GTA TAC CCA AAG ACA-3'). The PCR products were purified using a DNA extraction kit from agarose gel. The PCR products were then cloned into a pIRES2-EGFP expression vector (Clontech Laboratories INC) having CMV promoter for expression in cells as described above.

### Construction of the present deletion nucleotide sequence linked to MIF (macrophage inhibitory factor,) or YFP(yellow fluorescent protein

PCRs were performed using a plasmid MIF-YFPNI comprising MIF, or pEYFP-Nl expression vector (Clontech Laboratories INC.) comprising YFP as a template to construct the present deletion nucleotide sequence linked to MIF or YFP and the following primers having at each end Xho I and EcoRI restriction site, respectively for subsequent cloning: **Forward primer:** XhoI MIFF(5'-TTC TCG ATA TG CCG ATG TTC ATC GTA AA-3'), XhoI ins-MIFF(5'-TTC TCG ATA CCT CGA CAA GGC ATG CCG ATG TTC ATC GTA AA-3'), **Reverse Primer:** EcoRI MIFR(5'-AAG AAT TCC CAG GCG AAG GTG GAG TTGT-3'). The PCR products were purified using a DNA extraction kit from agarose gel. The PCR products were then cloned into a pEYFP-N1 expression vector (Clontech Laboratories INC) having CMV promoter for expression in cells as described above.

### Cell culture

Cell lines NIH-3T3(mouse fibroblast cell) originated from mouse, COS7(monkey kidney fibroblast cell) originated from monkey and HEK293(human kidney fibroblast cell) originated from human were cultured in DMEM (Hyclon), and Huh7(Human hepatocellular carcinoma) cell in RPMI 1640 (Hyclon), each medium containing 10% Bovine serum (Invitrogen Co., Carlsbad, CA, USA) and 2 mmol/ml L-glutamin, 100 µg/ml penicillin and 100 units/ml of streptomycing at 37°C and 5% CO2.

### Determination of luciferase activity

Cells were seeded to each of a 24 well plate at 2x10⁵ cells and 1µg of plasmid of interest was transiently transfected onto the cells in each well using Lipofectamin 2000 (Invtrogen Co) according to the manufacturer's instruction. After 48 hrs, PLB (Promega, USA) was added to each well to lyse the cells and luciferase activity was measured using Dual-luciferase reagent (Promega) and luminometer(Turner BioSystem, USA). Twenty ng of Renilla plasmid was cotransfected to normalize the variation in tranfection efficiency.

### Fluorescent microscopy

Cells were seeded to each of a 24 well plate at 2x10⁵ cells and 4µg of plasmid of interest abd beta-galactosidase expreession plasmid for normalization was transiently transfected onto the cells in each well using Lipofectamin 2000 (Invtrogen Co) according to the manufacturer's instruction. After 72 hrs, the cells expressing EGFP were examined using a fluorescent microscope.

### RT-PCR (Reverse Transcriptase Polymerase Chain Reaction)

Total RNA was extracted from the cells transfected as described above using RneasyPlus Mini kit(QIAGEN Inc.), which was then used for reverse transcription to synthesize cDNA using SuperScript™ II Reverse Transcriptase(Invitrogen Co) at 42°C for 60 min followed by 10 minute incubation at 72°C. Then 50ng of cDNA was used as a template for PCR to examine HBV surface antigen expression at mRNA level using the following primes: as HBV primers: Forward primer : Hepa p7 (5'- GTG GTG GAC TTC TCT CAA TTT TC - 3') and Reverse primer: Hepa p8 (5'- CGG TAW AAA GGG ACT CAM GAT - 3') and as GFP primers: Forward primer: 5'-ATG GTG AGC AAG GGC GAG GA-3') and Reverse primer: 5'-TTA CTT GTA CAG CTC GTC CA-3').

### Quantitative Polymerase Chain Reaction (Q-PCR)

The RNA was extracted and cDNA was synthesized as described above, which was then used for real time PCR using SYBR. The primers used were as follows: For luciferase detection: luci-RE-F (5'- TGC CCG CGA CCC CAT CT-3'), luci-RE-R (5'- CCC GAA AGC CGC AGA TCA AGT AGC - 3'), for Neomycin for quantitation: RE-NeoF (5'- TGG AGA GGC TAT TCG GCT ATG-3') and RE-NeoR (5' - TGC CTC GTC TTG CAG TTC ATT-3'). Specifically 12.5µl SYBR Green master mix (Bioline, UK), 1.25µl SF-Real time primer, 1.25ul SR-Real time primer, 9µl of sterilized distilled water and 1µl cDNA were added to each well of 384 well plate and real time PCR was performed using ABI 7900 (Applied Biosystems, USA) under the following condition: 10 min at 45 °C, 95 °C 2 min, 40 cycles of 95 °C 5sec, 60 °C 10sec and 72 °C 5sec. For melting curve analysis, 95 °C 10sec, 53 °C 30 sec reaction followed by increase of the temperature from 53 °C to 90 °C by 0.1 °C per sec. All the experiments were performed triplicate.

### Western blot

The cells transfected as described above were washed twice with PBS and harvested in PBS as cell pellet. The cells were then resuspened in a lysis buffer (50 mM Tris pH7.5, 10 mM EDTApH8.0, 1 mM PMSF, 2 mM NaF, 2 mM Na3VO4, 0.1% NP 40, 100 g/*µℓ* Leupeptin, 100 g/*µℓ* and Aprotinin) and lysed by sonification treating the cells twice for 15sec each with Sonicator Ultrasomic processor Misonix, USA) on ice, which was then centrifuged at 4°C at 13,000rpm for 10min. The supernatant was collected and used for subsequent experiment. The protein concentration was measured using Bradford Protein Assay(Bio-Rad, CA, USA) and 30µg of protein was loaded onto each well of SDS-PAGE and separated by electrophoresis and the gel was transferred to Nitrocellulosemembrane (Amersham International plc, UK) for immunoblotting. The first antibody used was monoclonal anti-preSl(Aprogen Daejeon, Korea), monoclonal anti-HBs(Tokyo Future Style Inc., Ibaraki,Japan), polyclonal monoclonal-anti-GFP, polyclonal monoclonal-anti-MIF, anti-β-actin (Santa Cruz Biotechnoolgy INC., CA, USA) at 1:1000 dilution and antibody labelled with horseradish peroxidase was used as a secondary antibody which was then analyzed using SuperSignal West Pico Chemiluminescence Substrate (Pierce, USA) and ImageQuant™ LAS 4000 Mini Biomolecular Imager(GE Healthcare Bio-Sciences, Sweden). Multi Gague V 3.0(Fuji Film, Japan) was used for densitometry and GFP and β-actin were used for normalization.

### Results

### The Effect of the present cis-acting element linked to HBV preS1 promoter on the luciferase activity.

Each of the cis acting element with a 21 nucleotide deletion in the middle represented by SEQ ID NO: 1, the cis acting element with a 21 nucleotide deletion at the starting region represented by SEQ ID NO: 2 and the cis acting element with a 15 nucleotide deletion in the middle represented by SEQ ID NO: 3, which were deletion type specific to Korean patient with severe liver disease, was cloned into pGL4.10 vector. The vector comprising each of the cis-acting element was then transfected into HEK293,Huh7 and NIH3T3 cells and the luciferase activity was measured from each cell line. As shown in FIG. 2, the deletion type of SEQ ID NO:2 with a deletion at the starting region has increase the luciferase activity by about 3.3 times in HEK293, by about 3.2 times in Huh7 and by about 2.1 times in NIH3T3 compared to the vector only containing preS1 promoter.

### The Effect of the present cis-acting element linked to various promoters on the luciferase activity

The following results are found in FIGs 3 to 6, To determine the nucleotides which are important for increasing the translational efficiency in the present cis acting element, various constructs having a mutation in the present cis acting element as described below were made by cloning the element into a pIRES2-EGFP vector comprising cytomegalovirus promoter, which were then transfected into HEK293, Huh7 or NIH3T3 cells followed by measuring luciferase activity. As a result, 13 nucleotide sequence represented by SEQ ID NO: 5 was found to have the highest activity, which increased the luciferase activity by about 2.4 times in HEK293, by about 3.6 times in Huh7 and by about 3.1 times in NIH3T3. Further A to G substitution mutation at the fifth nucleotide from the 3' end of the 13 nucleotide sequence represented by SEQ ID NO: 12 was found to have increased the luciferase activity by about 3.1 times in HEK293, by about 3.1 times in Huh7 and by about 1.9 times in NIH3T3. And the C to T substitution at mutation at the first nucleotide from the 3' end of the 13 nucleotide sequence represented by SEQ ID NO: 8 and the G to A substitution at the second nucleotide from the 3' end of the 13 nucleotide sequence represented by SEQ ID NO: 9 resulted in zero luciferase activity, which was due to the fact that the luciferase could not be translated from the normal ATG site due to such substitutions.

Further, other vectors with CMV promoter were also examined in CT-GFP vector system. For this, 20 or 13 mer nucleotide sequence (SEQ ID NO:5) and 13 mer sequence with C to T substitution (SEQ ID NO: 8) and G to A substitution (SEQ ID NO:9) in the context with CT-GFP vector was examined for the luciferase activity. As a result, the 13 nucleotide sequence represented by SEQ ID NO: 5 increased the luciferase activity by about 2.7 times in HEK293, by about 2.4 times in Huh7 and by about 2.2 times in NIH3T3 compared to the control luciferase. The C to T substitution mutation (SEQ ID NO: 8), which had no activity in pIRES2-EGFP system, showed the luciferase activity comparable to the control luciferase. The G to A substitution mutation (SEQ ID NO: 9) showed no activity.

Also similar experiments were done in pEF6/V5-HIS vector system comprising human elongation 1-alhpa promoter, the elements as used in CT-GFP vector system. As a result, the 13 nucleotide sequence represented by SEQ ID NO: 5 increased the luciferase activity by about 3.8 times in HEK293, by about 2.7 times in Huh7 and by about 2.5 times in NIH3T3 compared to the control luciferase. The C to T substitution mutation (SEQ ID NO: 8), which had no activity in pIRES2-EGFP system, showed the luciferase activity comparable to the control luciferase. The G to A substitution mutation (SEQ ID NO: 9) showed only weak activity in NIH3T3 cells and no activity at all in other cells.

### The Effect of the present cis-acting element linked to a promoter on the luciferase activity at mRNA level.

The constructs prepared as above for the luciferase activity were used for Quantitative real-time PCR to compare the luciferase activity at mRNA level. For this, each of the plasmid construct was transfected into eukaryotic cells Huh7 (human hepatoma cell line) and CHO (hamster ovarian cell line) and the total RNA was extracted using RNA extraction kit (Qiagen) and cDNA was synthesized. Then qPCR was performed on the cDNA. As a result, the 13 mer nucleotide sequence showed the highest activity and other elements with substitution mutation showed little or no activity as shown in FIG. 7.

### The Effect of the present cis-acting element linked to a promoter on the luciferase activity in the cell lines originated from various species.

The present cis acting element found in HBV virus was tested in cell lines originated from various species. Each of the plasmid comprising the control luciferase without the present element, or the luciferase linked to the 13 mer nucleotide element (SEQ ID NO: 5) was transfected into Jurkat (human T-lymphocyte cell line), THP1 (human mononuclear cell line), cells derived from mouse bone marrow, human peripheral blood mononuclear cell, mouse mesenchymal stem cell, and human mesenchymal stem cell and the luciferase activity was measured from each cell transfected. As a result, the 13 mer nucleotide represented by SEQ ID NO: 5 was found to have the highest activity, which increased the luciferase activity by about 4.3 times in Jurkat, by about 2.8 times in THP1, by about 2.9 times in the cells from mouse bone marrow, by about 6 times in the cells from human peripheral blood mononuclear cells, by about 1.8 times in the cells from mouse mesenchymal stem cells and by about 4 times in the cells from human mesenchymal stem cells compared to the control as shown in FIG. 8.

### Determination of the luciferase expression level using RT-PCR and western blot.

The results are shown in FIGs. 9, 11, 13 and 15. The pIRES-EGFP vector comprising deletion mutation in preS1 starting region (SEQ ID NO: 14) and wild type large surface antigen gene, large surface antigen gene transcribed from ATG which is located 33 nucleotide downstream the wild type gene, and wild type small surface antigen gene or 20mer nucleotide element (SEQ ID NO: 4) was transfected into appropriate cell lines and the expression level of the surface antigen was examined from each cell line transfected at mRNA level using RT-PCR. As a result, wild type large surface antigen and variant large surface antigen (SEQ ID NO:14) and the one with 33 nt deletion were all found to have no changes in the surface antigen expression at mRNA level as shown in FIG. 14. Also the same result was obtained with small surface antigen linked to 20mer element (SEQ ID NO: 4) as shown in FIG. 14.

Also when the same experiment was done in HEK293 and Huh7 cells to examine the expression level of surface antigen at the protein level using western blot, compared to the wild type large surface antigen, the expression of the large surface antigen comprising the deletion mutation (SEQ ID NO: 14) was found to be increased by about 2 times in HEK293 and by about 2.8 times in Huh7 cells. Also when the same experiment done with small surface antigen, it was found that the expression of the small surface antigen linked to the 20mer element (SEQ ID NO: 4) was increased by about 1.6 times in HEK293 and by about 2.9 times in Huh7 cells.

### Determination of the EGFP expression level under CMV promoter

The wild type EGFP gene or the EGFP gene linked to the 13mer element was cloned into a fusion vector CT-GFP with CMV promoter. The constructed plasmid was then transfected into Huh7 or NIH3T3 cells and the expression of GFP was examined using a fluorescent microscopy. As a result, EGFP gene linked to the 13mer nucleotide (SEQ ID NO:5) was found to be expressed more in both cell lines compared to the EGFP without the 13mer nucleotide. Also, the 13mer element linked to EGFP in the context of pcDNA 3.3-TOPO with CMV promoter when it was transfected into COS7 cell line and analyzed by western blot was found to increase the protein expression by about 3.7 times compared to the EGFP without the 13mer nucleotide as shown in FIG. 10.

### Determination of the expression of HBV large surface antigen and small surface antigen.

Results are shown in FIGs 12 to 15. pIRES-EGFP vector comprising deletion mutation in preS1 starting region (SEQ ID NO: 14), wild type large surface antigen gene, large surface antigen gene transcribed from ATG which is located 33 nucleotide downstream the wild type gene, and wild type small surface antigen gene or 20mer nucleotide element (SEQ ID NO: 4) was transfected into HEK293 and Huh7 cells and the expression level of the surface antigen was examined from each cell line transfected at mRNA level using RT-PCR. As a result, wild type large surface antigen and variant large surface antigen (SEQ ID NO:14) and the one with 33 nt deletion were all found to have no changes in the surface antigen expression at mRNA level. This indicates that the cis-acting element derived from HBV having a deletion mutation in preS1 starting region increases the protein expression at the translational level without affecting the transcriptional level.

Also, when the same experiment was done in HEK293 and Huh7 cells and the immunoblotting was done to examine the surface antigen expression at the translational level. As a result, the expression of the large surface antigen comprising the deletion mutation (SEQ ID NO: 14) was increased by about 2 times in HEK293 and by about 2.8 in Huh7 cells compared to the control large surface antigen. Also the expression of the small surface antigen comprising the 20 mer nucleotide deletion mutation (SEQ ID NO: 4) was increased by about 1.6 times in HEK293 and by about 2.9 in Huh7 cells compared to the control large surface antigen.

### The Effect of the present cis-acting element linked to MIF-YFP on the expression of MIF gene

The present 13mer cis acting element (SEQ ID NO: 5) linked to MIF (Macrophage Inhibitory Factor), which was then cloned into pEYFP-N1(Clontech) comprising YFP to construct a vector expressing MIF-YFP under the control of the present 13mer element. As a control, vector comprising MIF-YFP without the 13mer element was also cloned. The constructs were then transfected to appropriate cells and the expression of MIF-YFP at the translational level was examined by immuno blotting using MIF antibody and YFP antibody. As a result the 13mer nucleotide (SEQ ID NO: 5) increased the expression level of MIF by about 2.9 times and GFP by about 3 times as shown in FIG. 15

### The detection of the deleted region in preS1 using a nested PCR

PCR was performed to obtain a fragment of 1491 bp in length from 2814 to 989 (genotype C2 HBV Genbank assession no. AY641558) using as a template the DNA from a patient and the following primers: Forward primer: DelF2 (5'-GGG TCA CCA TAT TCT TGGG-3') and Reverse primer: HB2R (5'-CAT ACT TTC CAA TCA ATA GG-3'). The condition was as follows: first denaturation: 95°C 10min; 30 cycle: 95°C 45 sec, 58°C 60sec, 72°C 90 sec; final extension: 72°C 5min. The second PCR was performed on the fragment synthesized in the first PCR using the following primers: Forward primer: Del-PRA-F1 (50-CTT GGG AAC AAG AGCTACAGC-30) and Reverse primer: preS-R1 (50- TTG AGA GAA GTC CAC CAC GA-30) to obtain a fragment of 663-bp from 2827 to 274.

As described and demonstrated hereinbefore, the present cis-acting element is able to increase the protein expression of HBV surface antigen. In addition the cis acting element is able to increase the protein expression under a variety of promoters in various cell lines originated from various species. Thus the present cis acting element is useful for the protein production.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

## Claims

1. An isolated cis-acting element for eukaryotic cells selected from the group consisting of polynucleotides SEQ ID NO: 5, SEQ ID NO: 4, SEQ ID NO: 6, SEQ ID NO: 2, SEQ ID NO: 11, SEQ ID NO: 12, SEQ ID NO: 13, SEQ ID NO: 19, and SEQ ID NO: 20.

2. The isolated cis-acting element of Claim 1, wherein the element is located within 30 base pair 5' upstream of a translational initiation codon.

3. The isolated cis-acting element of Claim 1 or 2, wherein the element is located at 3' side of a promoter that is operable in the eukaryotic cells.

4. A vector for expression in eukaryotic cells comprising a promoter operable in eukaryotic cells and a cis-acting element for eukaryotic cells selected from the group consisting of polynucleotides SEQ ID NO: 5, SEQ ID NO: 4, SEQ ID NO: 6, SEQ ID NO: 2, SEQ ID NO: 11, SEQ ID NO: 12, SEQ ID NO: 13, SEQ ID NO: 19, and SEQ ID NO: 20.

5. The vector of Claim 4, further comprising a nucleotide sequence encoding a protein of interest.

6. The vector of Claim 5, wherein the promoter that is operable in eukaryotic cells includes a HBV(Hepatitis B Virus) promoter, a CMV(Cytomegalovirus) promoter, an Adenovirus late promoter, a Vaccinia virus 7.5K promoter, a SV40(Simian Virus 40) promoter, a SV40E1 promoter, a tk promoter of HSV(Herpes simplex virus), a RSV(Respiratory syncytial virus) promoter, an EF1(elongation factor-1 alpha) promoter, a metallothionein promoter, a beta actin promoter, a promoter of human IL-2(interleukin-2) gene, a promoter of human IFN(interferon) gene, a promoter of human IL-4(interleukin-4) gene, a promoter of human lymphotoxin gene or a promoter of human GM-CSF(Granulocyte macrophage colony-stimulating factor).

7. The vector of Claim 4, wherein the eukaryotic cell is an animal cell originated from a mammal, a rodent, or an insect.

8. The vector of Claim 5 wherein the protein is a virus antigen, a fluorescent protein, a hormone, a cytokine, an antibody, a peptide aptamer, a Adnectin, an affibody, an Avimer or a Kunitz domain.

9. A cell comprising the vectors according to any one of Claims 4 to 8.

10. The cell of Claim 9, wherein the eukaryotic cell is a CHO(Chinese hamster ovary) cell, HEK293(human embryonic kidney) cell, a Huh7 cell, a NIH3T3 cell, a VERO cell, a HeLa cell, a WI38 cell, a BHK(Baby hamster kidney) cell, a COS cell or a MDCK(Madin-Darby Canine Kidney) cell.

11. A method of producing a recombinant protein comprising a step of culturing the animal cell according to Claim 9 or 10.

12. A method of producing a protein from an exogenous gene in a eukaryotic cell, comprising a step of introducing a vector which comprises the cis-acting element according to any one of Claims 1 to 3 and a promoter and an exogenous gene operably linked to the promoter.

## Patentansprüche

1. Isoliertes cis-wirkendes Element für eukaryotische Zellen, das aus der Gruppe gewählt ist, die aus den Polynucleotiden SEQ ID NO: 5, SEQ ID NO: 4, SEQ ID NO: 6, SEQ ID NO: 2, SEQ ID NO: 11, SEQ ID NO: 12, SEQ ID NO: 13, SEQ ID NO: 19 und SEQ ID NO: 20 besteht.

2. Isoliertes cis-wirkendes Element nach Anspruch 1, wobei sich das Element in 30 Basispaar 5' stromaufwärts eines Translationsinitiationscodons befindet.

3. Isoliertes cis- wirkendes Element nach Anspruch 1 oder 2, wobei sich das Element auf der 3'-Seite eines Promoters befindet, der in den eukaryotischen Zellen betreibbar ist.

4. Vektor zur Expression in eukaryotischen Zellen, der einen Promoter, der in eukaryotischen Zellen betreibbar ist und ein cis-wirksames Element für eukaryotische Zellen, das aus der Gruppe gewählt ist, die aus den Polynucleotiden SEQ ID NO: 5, SEQ ID NO: 4, SEQ ID NO: 6, SEQ ID NO: 2, SEQ ID NO: 11, SEQ ID NO: 12, SEQ ID NO: 13, SEQ ID NO: 19 und SEQ ID NO: 20 besteht, umfasst.

5. Vektor nach Anspruch 4, der ferner eine Nukleotidsequenz umfasst, die ein Protein von Interesse codiert.

6. Vektor nach Anspruch 5, wobei der Promoter, der in eukaryotischen Zellen betreibbar ist, einen HBV-Promoter (Hepatitis B-Virus-Promoter), einen CMV-Promoter (Zytomegalievirus-Promoter), eine späten Adenovirus-Promoter, einen Vaccinavirus 7,5K-Promoter, einen SV40-Promoter (Simianvirus 40-Promoter), einen SV40E1-Promoter, einen TK-Promoter für das HSV (Herpes-simplex-Virus), einen RSV-Promoter (Promoter für das respiratorische Synzytialvirus), einen EF1-Promoter (Dehnungsfaktor-1 alpa-Promoter), einen Metallothionein-Promoter, eine Beta-Actin-Promoter, einen Promoter für ein menschliches IL-2-Gen (Interleukin-2-Gen), einen Promoter für ein menschliches IFN-Gen (Interferon-Gen), einen Promoter für ein menschliches IL-4-Gen (Interleukin-4-Gen), einen Promoter für ein menschliches Lymphotoxin-Gen oder einen Promoter für einen menschlichen GM-CSF (Granulozyten-Makrophagen-Koloniestimulierungsfaktor) enthält.

7. Vektor nach Anspruch 4, wobei die eukaryotische Zelle eine Tierzelle ist, die von einem Säugetier, einem Nagetier oder einem Insekt stammt.

8. Vektor nach Anspruch 5, wobei das Protein ein Virusantigen, ein fluoreszierendes Protein, ein Hormon, ein Zytokin, ein Antikörper, ein Peptid-Aptamer, ein Adnektin, ein Affibody, ein Avimer oder eine Kunitz-Domäne ist.

9. Zelle, die die Vektoren nach einem der Ansprüche 4 bis 8 umfasst.

10. Zelle nach Anspruch 9, wobei die eukaryotische Zelle eine CHO-Zelle (Eierstockzelle eines chinesischen Hamsters), eine HEK293-Zelle (menschliche Embryonierenzelle), eine Huh7-Zelle, eine NIH3T3-Zelle, eine VERO-Zelle, eine HeLa-Zelle, eine WI38-Zelle, eine BHK-Zelle (Babyhamsternierenzelle), eine COS-Zelle oder eine MDCK-Zelle (Madin-Darby Hundenierenzelle) ist.

11. Verfahren zum Herstellen eines rekombinanten Proteins, das einen Schritt des Züchtens der Tierzelle nach Anspruch 9 oder 10 umfasst.

12. Verfahren zum Herstellen eines Proteins aus einem exogenen Gen in einer eukaryotischen Zelle, das einen Schritt des Einführens eines Vektors, der das cis-wirkende Element nach einem der Ansprüche 1 bis 3 und einen Promoter und ein exogenes Gen, das funktionsfähig mit dem Promoter verbunden ist, umfasst.

## Revendications

1. Élément agissant en cis isolé pour les cellules eucaryotes sélectionné dans le groupe constitué des polynucléotides SEQ ID NO : 5, SEQ ID NO : 4, SEQ ID NO : 6, SEQ ID NO : 2, SEQ ID NO : 11, SEQ ID NO : 12, SEQ ID NO : 13, SEQ ID NO : 19 et SEQ ID NO : 20.

2. Élément agissant en cis isolé selon la revendication 1, dans lequel l'élément se trouve dans les 30 paires de bases en 5' en amont d'un codon d'initiation de la traduction.

3. Élément agissant en cis isolé selon la revendication 1 ou 2, dans lequel l'élément se trouve au niveau du côté 3' d'un promoteur qui peut fonctionner dans les cellules eucaryotes.

4. Vecteur pour l'expression dans des cellules eucaryotes comprenant un promoteur pouvant fonctionner dans des cellules eucaryotes et un élément agissant en cis pour des cellules eucaryotes sélectionné dans le groupe constitué des polynucléotides SEQ ID NO : 5, SEQ ID NO : 4, SEQ ID NO : 6, SEQ ID NO : 2, SEQ ID NO : 11, SEQ ID NO : 12, SEQ ID NO : 13, SEQ ID NO : 19 et SEQ ID NO : 20.

5. Vecteur selon la revendication 4, qui comprend en outre une séquence nucléotidique codant pour une protéine d'intérêt.

6. Vecteur selon la revendication 5, dans lequel le promoteur qui peut fonctionner dans les cellules eucaryotes comprend un promoteur du virus de l'hépatite B (HBV), un promoteur du CMV (cytomégalovirus), un promoteur tardif d'adénovirus, un promoteur 7.5K du virus de la vaccine, un promoteur du SV40 (virus simien 40), un promoteur du SV40E1, un promoteur tk du VHS (virus de l'herpès simplex), un promoteur du VRS (virus respiratoire syncytial), un promoteur d'EF1-α (facteur d'élongation 1), un promoteur de la métallothionéine, un promoteur de la β-actine, un promoteur du gène de l'IL-2 (interleukine 2) humaine, un promoteur du gène de l'IFN (interféron humain), un promoteur du gène de l'IL-4 (interleukine 4) humaine, un promoteur du gène de la lymphotoxine humaine ou un promoteur du gène du GM-CSF (facteur de stimulation des colonies de granulocytes et de macrophages) humain.

7. Vecteur selon la revendication 4, dans lequel la cellule eucaryote est une cellule animale provenant d'un mammifère, d'un rongeur, ou d'un insecte.

8. Vecteur selon la revendication 5, dans lequel la protéine est un antigène viral, une protéine fluorescente, une hormone, une cytokine, un anticorps, un aptamère peptidique, une adnectine, un afficorps, un avimère ou un domaine de Kunitz.

9. Cellule comprenant les vecteurs selon l'une quelconque des revendications 4 à 8.

10. Cellule selon la revendication 9, dans laquelle la cellule eucaryote est une cellule CHO (ovarienne de hamster chinois), une cellule HEK293 (rénale embryonnaire humaine), une cellule Huh7, une cellule NIH3T3, une cellule VERO, une cellule HeLa, une cellule WI38, une cellule BHK (rénale de hamster nouveau-né), une cellule COS, ou une cellule MDCK (rénale canine Madin-Darby).

11. Procédé de production d'une protéine recombinante comprenant une étape de culture de la cellule animale selon la revendication 9 ou 10.

12. Procédé de production d'une protéine à partir d'un gène exogène dans une cellule eucaryote, comprenant une étape d'introduction d'un vecteur qui comprend l'élément agissant en cis selon l'une quelconque des revendications 1 à 3 et un promoteur et un gène exogène fonctionnellement lié au promoteur.


### Sequence Listing

<110> SNU RDB Foundation
<120> Cis acting regulatory elements from HBV and its use
<130> PCT201210002P
<150> 2011-0124053
   <151> 2011-11-25
<160> 33
<170> KopatentIn 2.0
<210> 1
   <211> 12
   <212> DNA
   <213> Hepatitis B virus
<400> 1
   atgggacaag gc 12
<210> 2
   <211> 11
   <212> DNA
   <213> Hepatitis B virus
<400> 2
   ctcgacaagg c 11
<210> 3
   <211> 18
   <212> DNA
   <213> Hepatitis B virus
<400> 3
   accaaacctc gacaaggc 18
<210> 4
   <211> 20
   <212> DNA
   <213> Hepatitis B virus
<400> 4
   gcaccaaacc tcgacaaggc 20
<210> 5
   <211> 13
   <212> DNA
   <213> Hepatitis B virus
<400> 5
   acctcgacaa ggc 13
<210> 6
   <211> 9
   <212> DNA
   <213> Hepatitis B virus
<400> 6
   cgacaaggc 9
<210> 7
   <211> 30
   <212> DNA
   <213> Hepatitis B virus
<400> 7
   gcaccaaacc gcaccaaacc tcgacaaggc 30
<210> 8
   <211> 13
   <212> DNA
   <213> Hepatitis B virus
<400> 8
   acctcgacaa ggt 13
<210> 9
   <211> 13
   <212> DNA
   <213> Hepatitis B virus
<400> 9
   acctcgacaa gac 13
<210> 10
   <211> 13
   <212> DNA
   <213> Hepatitis B virus
<400> 10
   acctcgacaa agc 13
<210> 11
   <211> 13
   <212> DNA
   <213> Hepatitis B virus
<400> 11
   acctcgacag ggc 13
<210> 12
   <211> 13
   <212> DNA
   <213> Hepatitis B virus
<400> 12
   acctcgacga ggc 13
<210> 13
   <211> 13
   <212> DNA
   <213> Hepatitis B virus
<400> 13
   acctcgataa ggc 13
<210> 14
   <211> 11
   <212> DNA
   <213> Hepatitis B virus
<400> 14
   ctcgataagg c 11
<210> 15
   <211> 6
   <212> DNA
   <213> Hepatitis B virus
<400> 15
   caaggc 6
<210> 16
   <211> 3
   <212> DNA
   <213> Hepatitis B virus
<400> 16
   ggc 3
<210> 17
   <211> 13
   <212> DNA
   <213> Hepatitis B virus
<400> 17
   acctcgcaaa ggc 13
<210> 18
   <211> 13
   <212> DNA
   <213> Hepatitis B virus
<400> 18
   acctcgaaaa ggc 13
<210> 19
   <211> 13
   <212> DNA
   <213> Hepatitis B virus
<400> 19
   acctcgacac ggc 13
<210> 20
   <211> 13
   <212> DNA
   <213> Hepatitis B virus
<400> 20
   acctcgaaca ggc 13
<210> 21
   <211> 13
   <212> DNA
   <213> Hepatitis B virus
<400> 21
   acctcgacaa cgc 13
<210> 22
   <211> 17
   <212> DNA
   <213> Artificial Sequence
<220>
   <223> Primer for luciferase gene amplification- forward
<220>
   <221> misc_feature
   <222> (1)..(17)
   <223> primer
<400> 22
   tgcccgcgac cccatct 17
<210> 23
   <211> 24
   <212> DNA
   <213> Artificial Sequence
<220>
   <223> Primer for luciferase gene amplification
<220>
   <221> misc_feature
   <222> (1)..(24)
   <223> reverse
<400> 23
   cccgaaagcc gcagatcaag tagc 24
<210> 24
   <211> 21
   <212> DNA
   <213> Artificial Sequence
<220>
   <223> Primer for neomycin gene amplification
<220>
   <221> misc_feature
   <222> (1)..(21)
   <223> forward
<400> 24
   tggagaggct attcggctat g 21
<210> 25
   <211> 21
   <212> DNA
   <213> Artificial Sequence
<220>
   <223> Primer for neomycin gene amplification
<220>
   <221> misc_feature
   <222> (1)..(21)
   <223> reverse
<400> 25
   tgcctcgtct tgcagttcat t 21
<210> 26
   <211> 23
   <212> DNA
   <213> Artificial Sequence
<220>
   <223> Primer for HepaP7 gene amplification
<220>
   <221> misc_feature
   <222> (1)..(23)
   <223> forward
<400> 26
   gtggtggact tctctcaatt ttc 23
<210> 27
   <211> 21
   <212> DNA
   <213> Artificial Sequence
<220>
   <223> Primer for HepaP7 gene amplification
<220>
   <221> misc_feature
   <222> (1)..(21)
   <223> reverse
<400> 27
   cggtawaaag ggactcamga t 21
<210> 28
   <211> 20
   <212> DNA
   <213> Artificial Sequence
<220>
   <223> Primer for GFP gene amplification
<220>
   <221> misc_feature
   <222> (1)..(20)
   <223> forward
<400> 28
   atggtgagca agggcgagga 20
<210> 29
   <211> 20
   <212> DNA
   <213> Artificial Sequence
<220>
   <223> Primer for GFP gene amplification
<220>
   <221> misc_feature
   <222> (1)..(20)
   <223> reverse
<400> 29
   ttacttgtac agctcgtcca 20
<210> 30
   <211> 34
   <212> DNA
   <213> Artificial Sequence
<220>
   <223> Primer for detecting DT-2 type
<220>
   <221> misc_feature
   <222> (1)..(34)
   <223> DelF2, forward
<400> 30
   gggtcaccat attcttggga acaagagcta cagc 34
<210> 31
   <211> 20
   <212> DNA
   <213> Artificial Sequence
<220>
   <223> Primer for detecting DT-2 type
<220>
   <221> misc_feature
   <222> (1)..(20)
   <223> HB2R, reverse
<400> 31
   catactttcc aatcaatagg 20
<210> 32
   <211> 21
   <212> DNA
   <213> Artificial Sequence
<220>
   <223> Primer for detecting DT-2 type
<220>
   <221> misc_feature
   <222> (1)..(21)
   <223> Del-PRA-F1, forward for nested PCR
<400> 32
   cttgggaaca agagctacag c 21
<210> 33
   <211> 20
   <212> DNA
   <213> Artificial Sequence
<220>
   <223> Primer for detecting DT-2 type
<220>
   <221> misc_feature
   <222> (1)..(20)
   <223> preS-R1, reverse for nested PCR
<400> 33
   ttgagagaag tccaccacga 20